(19) Europäisches Patentamt
European Patent Office
Office européen des brevets

(11) EP 2 141 237 A1

(12) EUROPEAN PATENT APPLICATION

(43) Date of publication:
**06.01.2010 Bulletin 2010/01**

(51) Int Cl.:
*C12N 15/62* (2006.01) *C12N 9/24* (2006.01)
*C12N 9/10* (2006.01) *C07K 16/18* (2006.01)

(21) Application number: **09000153.8**

(22) Date of filing: **08.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **03.07.2008 KR 20080064483**

(71) Applicant: **Mogam Biotechnology Research Institute**
**Giheung-gu**
**Yongin-si 446-799**
**Kyonggi do (KR)**

(72) Inventors:
- **Kim, Jung-Seob**
  **Yongin-si**
  **Gyeonggi-do 448-990 (KR)**
- **Moon, Jae-Hoon**
  **Gunpo-si**
  **Gyeonggi-do 435-757 (KR)**
- **Oh, Mee Sook**
  **Yongin-si**
  **Gyeonggi-do 446-741 (KR)**
- **Lee, Kong Ju**
  **Yongin-si**
  **Gyeonggi-do 448-130 (KR)**
- **Yoon, Yeup**
  **Gyeonggi-do 427-010 (KR)**

(74) Representative: **Goddar, Heinz J. et al**
**Forrester & Boehmert**
**Pettenkoferstrasse 20-22**
**80336 München (DE)**

Remarks:
Amended claims in accordance with Rule 137(2) EPC.

# (54) Method for reducing the fucose content of recombinant proteins

(57) The present invention relates to a method for reducing the fucose content of a recombinant protein, which comprises expressing in an animal cell the recombinant protein and FUCA1, an FUCA1 mutant, FUCA2, or a fragment of FUT8 localization domain; or with a fusion protein of a fragment of FUT8 localization domain and a fragment of FUCA1, a FUCA1 mutant or FUCA2. Therefore, the antibody expressed according to the method of the present invention exhibits a reduced fucose content in their Fc regions, which leads to the improvement in the therapeutic effect thereof.

FIG. 1

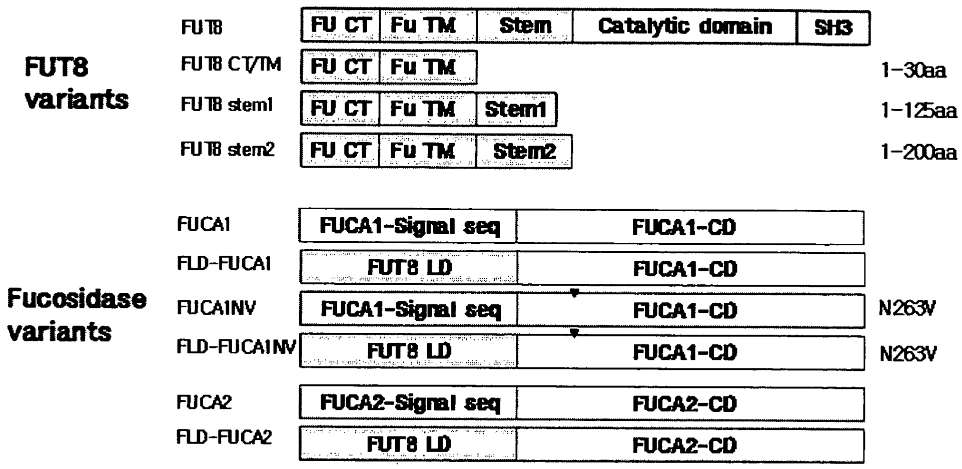


EP 2 141 237 A1

<110> MOGAM BIOTECHNOLOGY RESEARCH INSTITUTE

<120> METHOD FOR REDUCING FUCOSE CONTENTS OF RECOMBINANT PROTEINS

<130> PCA805035GCC

<160> 32

<170> KopatentIn 1.71

<210> 1
<211> 575
<212> PRT
<213> Homo sapiens

<400> 1

```
Met Arg Pro Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Pro Ala Ile
65                  70                  75                  80

Gly Arg Val Arg Val Leu Glu Glu Gln Leu Val Lys Ala Lys Glu Gln
                85                  90                  95

Ile Glu Asn Tyr Lys Lys Gln Thr Arg Asn Gly Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys Asn Leu Glu Gly Asn Glu
    130                 135                 140

Leu Gln Arg His Ala Asp Glu Phe Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Asp Trp Arg Glu Lys Glu Ala Lys Asp Leu Thr Glu Leu Val Gln
            180                 185                 190

Arg Arg Ile Thr Tyr Leu Gln Asn Pro Lys Asp Cys Ser Lys Ala Lys
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Ile
            260                 265                 270

Ser Thr Gly His Trp Ser Gly Glu Val Lys Asp Lys Asn Val Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Val Arg Val His
305                 310                 315                 320
```

```
Gly Asp Pro Ala Val Trp Trp Val Ser Gln Phe Val Lys Tyr Leu Ile
                325                 330                 335

Arg Pro Gln Pro Trp Leu Glu Lys Glu Ile Glu Glu Ala Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val
    370                 375                 380

His Val Glu Glu His Phe Gln Leu Leu Ala Arg Arg Met Gln Val Asp
385                 390                 395                 400

Lys Lys Arg Val Tyr Leu Ala Thr Asp Asp Pro Ser Leu Leu Lys Glu
                405                 410                 415

Ala Lys Thr Lys Tyr Pro Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Ile Tyr Ala
            500                 505                 510

His Gln Pro Arg Thr Ala Asp Glu Ile Pro Met Glu Pro Gly Asp Ile
        515                 520                 525

Ile Gly Val Ala Gly Asn His Trp Asp Gly Tyr Ser Lys Gly Val Asn
    530                 535                 540

Arg Lys Leu Gly Arg Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570                 575


<210>   2
<211>   30
<212>   PRT
<213>   Homo sapiens

<400>   2
Met Arg Pro Trp Thr Gly Ser Trp Arg Trp Ile Met Leu Ile Leu Phe
1               5                   10                  15

Ala Trp Gly Thr Leu Leu Phe Tyr Ile Gly Gly His Leu Val
            20                  25                  30


<210>   3
<211>   101
<212>   PRT
<213>   Homo sapiens

<400>   3
Met Arg Pro Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30
```

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Pro Ala Ile
65 70 75 80

Gly Arg Val Arg Val Leu Glu Glu Gln Leu Val Lys Ala Lys Glu Gln
85 90 95

Ile Glu Asn Tyr Lys
100

<210> 4
<211> 125
<212> PRT
<213> Homo sapiens

<400> 4
Met Arg Pro Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Pro Ala Ile
65 70 75 80

Gly Arg Val Arg Val Leu Glu Glu Gln Leu Val Lys Ala Lys Glu Gln
85 90 95

Ile Glu Asn Tyr Lys Lys Gln Thr Arg Asn Gly Leu Gly Lys Asp His
100 105 110

Glu Ile Leu Arg Arg Arg Ile Glu Asn Gly Ala Lys Glu
115 120 125

<210> 5
<211> 200
<212> PRT
<213> Homo sapiens

<400> 5
Met Arg Pro Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Pro Ala Ile
65 70 75 80

Gly Arg Val Arg Val Leu Glu Glu Gln Leu Val Lys Ala Lys Glu Gln
85 90 95

Ile Glu Asn Tyr Lys Lys Gln Thr Arg Asn Gly Leu Gly Lys Asp His
100 105 110

Glu Ile Leu Arg Arg Arg Ile Glu Asn Gly Ala Lys Glu Leu Trp Phe

115 120 125

Phe Leu Gln Ser Glu Leu Lys Lys Leu Lys Asn Leu Glu Gly Asn Glu
130 135 140

Leu Gln Arg His Ala Asp Glu Phe Leu Leu Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn
195 200

<210> 6
<211> 461
<212> PRT
<213> Homo sapiens

<400> 6
Met Arg Ser Arg Pro Ala Gly Pro Ala Leu Leu Leu Leu Leu Leu Phe
1 5 10 15

Leu Gly Ala Ala Glu Ser Val Arg Arg Ala Gln Pro Pro Arg Arg Tyr
20 25 30

Thr Pro Asp Trp Pro Ser Leu Asp Ser Arg Pro Leu Pro Ala Trp Phe
35 40 45

Asp Glu Ala Lys Phe Gly Val Phe Ile His Trp Gly Val Phe Ser Val
50 55 60

Pro Ala Trp Gly Ser Glu Trp Phe Trp Trp His Trp Gln Gly Glu Gly
65 70 75 80

Arg Pro Gln Tyr Gln Arg Phe Met Arg Asp Asn Tyr Pro Pro Gly Phe
85 90 95

Ser Tyr Ala Asp Phe Gly Pro Gln Phe Thr Ala Arg Phe Phe His Pro
100 105 110

Glu Glu Trp Ala Asp Leu Phe Gln Ala Ala Gly Ala Lys Tyr Val Val
115 120 125

Leu Thr Thr Lys His His Glu Gly Phe Thr Asn Trp Pro Ser Pro Val
130 135 140

Ser Trp Asn Trp Asn Ser Lys Asp Val Gly Pro His Arg Asp Leu Val
145 150 155 160

Gly Glu Leu Gly Thr Ala Leu Arg Lys Arg Asn Ile Arg Tyr Gly Leu
165 170 175

Tyr His Ser Leu Leu Glu Trp Phe His Pro Leu Tyr Leu Leu Asp Lys
180 185 190

Lys Asn Gly Phe Lys Thr Gln His Phe Val Ser Ala Lys Thr Met Pro
195 200 205

Glu Leu Tyr Asp Leu Val Asn Ser Tyr Lys Pro Asp Leu Ile Trp Ser
210 215 220

Asp Gly Glu Trp Glu Cys Pro Asp Thr Tyr Trp Asn Ser Thr Asn Phe
225 230 235 240

Leu Ser Trp Leu Tyr Asn Asp Ser Pro Val Lys Asp Glu Val Val Val
245 250 255

Asn Asp Arg Trp Gly Gln Asn Ser Ser Cys His His Gly Gly Tyr Tyr
260 265 270

Asn Cys Glu Asp Lys Phe Lys Pro Gln Ser Leu Pro Asp His Lys Trp
275 280 285

Glu Met Cys Thr Ser Ile Asp Lys Phe Ser Trp Gly Tyr Arg Arg Asp
290 295 300

Met Ala Leu Ser Asp Val Thr Glu Glu Ser Glu Ile Ile Ser Glu Leu
305 310 315 320

Val Gln Thr Val Ser Leu Gly Gly Asn Tyr Leu Leu Asn Ile Gly Pro
325 330 335

Thr Lys Asp Gly Leu Ile Val Pro Ile Phe Gln Glu Arg Leu Leu Ala
340 345 350

Val Gly Lys Trp Leu Ser Ile Asn Gly Glu Ala Ile Tyr Ala Ser Lys
355 360 365

Pro Trp Arg Val Gln Trp Glu Lys Asn Thr Thr Ser Val Trp Tyr Thr
370 375 380

Ser Lys Gly Ser Ala Val Tyr Ala Ile Phe Leu His Trp Pro Glu Asn
385 390 395 400

Gly Val Leu Asn Leu Glu Ser Pro Ile Thr Thr Ser Thr Thr Lys Ile
405 410 415

Thr Met Leu Gly Ile Gln Gly Asp Leu Lys Trp Ser Thr Asp Pro Asp
420 425 430

Lys Gly Leu Phe Ile Ser Leu Pro Gln Leu Pro Pro Ser Ala Val Pro
435 440 445

Ala Glu Phe Ala Trp Thr Ile Lys Leu Thr Gly Val Lys
450 455 460

<210> 7
<211> 467
<212> PRT
<213> Homo sapiens

<400> 7
Met Arg Pro Gln Glu Leu Pro Arg Leu Ala Phe Pro Leu Leu Leu Leu
1 5 10 15

Leu Leu Leu Leu Leu Pro Pro Pro Pro Cys Pro Ala His Ser Ala Thr
20 25 30

Arg Phe Asp Pro Thr Trp Glu Ser Leu Asp Ala Arg Gln Leu Pro Ala
35 40 45

Trp Phe Asp Gln Ala Lys Phe Gly Ile Phe Ile His Trp Gly Val Phe
50 55 60

Ser Val Pro Ser Phe Gly Ser Glu Trp Phe Trp Trp Tyr Trp Gln Lys
65 70 75 80

Glu Lys Ile Pro Lys Tyr Val Glu Phe Met Lys Asp Asn Tyr Pro Pro
85 90 95

Ser Phe Lys Tyr Glu Asp Phe Gly Pro Leu Phe Thr Ala Lys Phe Phe
100 105 110

Asn Ala Asn Gln Trp Ala Asp Ile Phe Gln Ala Ser Gly Ala Lys Tyr
115 120 125

Ile Val Leu Thr Ser Lys His His Glu Gly Phe Thr Leu Trp Gly Ser
130 135 140

Glu Tyr Ser Trp Asn Trp Asn Ala Ile Asp Glu Gly Pro Lys Arg Asp
145 150 155 160

Ile Val Lys Glu Leu Glu Val Ala Ile Arg Asn Arg Thr Asp Leu Arg
165 170 175

Phe Gly Leu Tyr Tyr Ser Leu Phe Glu Trp Phe His Pro Leu Phe Leu
180 185 190

Glu Asp Glu Ser Ser Ser Phe His Lys Arg Gln Phe Pro Val Ser Lys
195 200 205

Thr Leu Pro Glu Leu Tyr Glu Leu Val Asn Asn Tyr Gln Pro Glu Val
210 215 220

Leu Trp Ser Asp Gly Asp Gly Gly Ala Pro Asp Gln Tyr Trp Asn Ser
225 230 235 240

Thr Gly Phe Leu Ala Trp Leu Tyr Asn Glu Ser Pro Val Arg Gly Thr
245 250 255

Val Val Thr Asn Asp Arg Trp Gly Ala Gly Ser Ile Cys Lys His Gly
260 265 270

Gly Phe Tyr Thr Cys Ser Asp Arg Tyr Asn Pro Gly His Leu Leu Pro
275 280 285

His Lys Trp Glu Asn Cys Met Thr Ile Asp Lys Leu Ser Trp Gly Tyr
290 295 300

Arg Arg Glu Ala Gly Ile Ser Asp Tyr Leu Thr Ile Glu Glu Leu Val
305 310 315 320

Lys Gln Leu Val Glu Thr Val Ser Cys Gly Gly Asn Leu Leu Met Asn
325 330 335

Ile Gly Pro Thr Leu Asp Gly Thr Ile Ser Val Val Phe Glu Glu Arg
340 345 350

Leu Arg Gln Met Gly Ser Trp Leu Lys Val Asn Gly Glu Ala Ile Tyr
355 360 365

Glu Thr His Thr Trp Arg Ser Gln Asn Asp Thr Val Thr Pro Asp Val
370 375 380

Trp Tyr Thr Ser Lys Pro Lys Glu Lys Leu Val Tyr Ala Ile Phe Leu
385 390 395 400

Lys Trp Pro Thr Ser Gly Gln Leu Phe Leu Gly His Pro Lys Ala Ile
405 410 415

Leu Gly Ala Thr Glu Val Lys Leu Leu Gly His Gly Gln Pro Leu Asn
420 425 430

Trp Ile Ser Leu Glu Gln Asn Gly Ile Met Val Glu Leu Pro Gln Leu
435 440 445

Thr Ile His Gln Met Pro Cys Lys Trp Gly Trp Ala Leu Ala Leu Thr
450 455 460

Asn Val Ile
465

<210> 8
<211> 28
<212> DNA
<213> Artificial Sequence

<220>
<223> forward primer for FUCA1

<400> 8
ctagctagcc accatgaggt cgcggccg 28

<210> 9

<211> 35
<212> DNA
<213> Artificial Sequence

<220>
<223> reverse primer for FUCA1

<400> 9
ctactcgagc ttcactcctg tcagctttat agtcc 35

<210> 10
<211> 28
<212> DNA
<213> Artificial Sequence

<220>
<223> forward primer for FUCA2

<400> 10
ctagctagcc accatgcggc cccaggag 28

<210> 11
<211> 33
<212> DNA
<213> Artificial Sequence

<220>
<223> reverse primer for FUCA2

<400> 11
ctactcgagg atcacattag tcagggctag agc 33

<210> 12
<211> 36
<212> DNA
<213> Artificial Sequence

<220>
<223> forward primer for FUCA1 variant

<400> 12
ccatggtgac aggaacagac ctgaccccat cggtca 36

<210> 13
<211> 36
<212> DNA
<213> Artificial Sequence

<220>
<223> reverse primer for FUCA1 variant

<400> 13
tgaccgatgg ggtcaggtct gttcctgtca ccatgg 36

<210> 14
<211> 29
<212> DNA
<213> Artificial Sequence

<220>
<223> forward primer for FUT8

<400> 14
ctagctagcc accatgcggc catggactg 29

<210> 15
<211> 31
<212> DNA
<213> Artificial Sequence

<220>
<223> reverse primer for FUT8

<400> 15
ctactcgagt ttctcagcct caggatatgt g 31

<210> 16
<211> 34
<212> DNA
<213> Artificial Sequence

<220>
<223> primer for FUT8 localization domain fragment having 1 to 30 amino acid sequence

<400> 16
ctactcgagt accaagtgac cacctatata aaac 34

<210> 17
<211> 31
<212> DNA
<213> Artificial Sequence

<220>
<223> primer for FUT8 localization domain fragment having 1 to 125 amino acid sequence

<400> 17
ctactcgagc tctttagctc cattttcaat c 31

<210> 18
<211> 33
<212> DNA
<213> Artificial Sequence

<220>
<223> primer for FUT8 localization domain fragment having 1 to 200 amino acid sequence

<400> 18
ctactcgaga ttctgaagat atgttattct ccg 33

<210> 19
<211> 43
<212> DNA
<213> Artificial Sequence

<220>
<223> forward primer for FLD-FUCA1

<400> 19
gtagcggcgc ggaggctgct tgtaattttc aatctgttct ttg 43

<210> 20
<211> 43
<212> DNA
<213> Artificial Sequence

<220>
<223> reverse primer for FLD-FUCA1

<400> 20
caaagaacag attgaaaatt acaagcagcc tccgcgccgc tac 43

<210> 21
<211> 42
<212> DNA
<213> Artificial Sequence

<220>
<223> forward primer for FLD-FUCA2

<400> 21
aagcgcgtgg cgctgtgctt gtaattttca atctgttctt tg 42

<210> 22
<211> 42
<212> DNA
<213> Artificial Sequence

<220>
<223> reverse primer for FLD-FUCA2

<400> 22
caaagaacag attgaaaatt acaagcacag cgccacgcgc tt 42

<210> 23
<211> 43
<212> DNA
<213> Artificial Sequence

<220>
<223> forward primer for FLD-FUCA1NV

<400> 23
gtagcggcgc ggaggctgta ccaagtgacc acctatataa aac 43

<210> 24
<211> 43
<212> DNA
<213> Artificial Sequence

<220>
<223> reverse primer for FLD-FUCA1NV

<400> 24
gttttatata ggtggtcact tggtacagcc tccgcgccgc tac 43

<210> 25
<211> 19
<212> DNA
<213> Homo sapiens

<400> 25
gctgagtctc tccgaatac 19

<210> 26
<211> 65
<212> DNA
<213> Artificial Sequence

<220>

<223> forward primer for FUT8 siB

<400> 26
gatccgctga gtctctccga atacttcaag agagtattcg gagagactca gccatttttt 60

ggaaa 65

<210> 27
<211> 65
<212> DNA
<213> Artificial Sequence

<220>
<223> reverse primer for FUT8 siB

<400> 27
agcttttcca aaaaatggct gagtctctcc gaatactctc ttgaagtatt cggagagact 60

cagcg 65

<210> 28
<211> 22
<212> DNA
<213> Homo sapiens

<400> 28
gaacactcat catcttggaa tc 22

<210> 29
<211> 65
<212> DNA
<213> Artificial Sequence

<220>
<223> forward primer for FUT8 siR

<400> 29
gatccgaaca ctcatcttgg aatcttcaag agagattcca agatgagtgt tcgctttttt 60

ggaaa 65

<210> 30
<211> 64
<212> DNA
<213> Artificial Sequence

<220>
<223> reverse primer for FUT8 siR

<400> 30
agcttttcca aaaaagcgaa cactcatctt ggaatctctc ttgaagattc caagatgagt 60

gttc 64

<210> 31
<211> 27
<212> DNA
<213> Artificial Sequence

<220>
<223> forward primer for FcrRIIIa

<400> 31
ctagctagct ctttggtgac ttgtcca 27

<210> 32
<211> 48
<212> DNA
<213> Artificial Sequence

<220>
<223> reverse primer for FcrRIIIa

<400> 32
ctagttaact caatgatgat gatgatgatg cccaggtgga aagaatga 48

**Description**

## FIELD OF THE INVENTION

The present invention relates to a method for reducing the fucose content of a recombinant protein, which comprises expressing in an animal cell the recombinant protein and FUCA 1, an FUCA1 mutant, FUCA2, or a fragment of FUT8 localization domain; or with a fusion protein of a fragment of FUT8 localization domain and a fragment of FUCA1, a FUCA1 mutant or FUCA2.

## BACKGROUND OF THE INVENTION

With the completion of human genome project and the identification of numerous disease-related genes through post-genomic approaches, therapeutic protein research has undergone an enormous progress. Most therapeutic proteins are glycoproteins whose therapeutic effects had been previously known to be affected only by their amino acid sequences. However, it has recently been established that the activity of a therapeutic protein can be effectively improved by modifying the sugar chain attached thereto, and such a sugar chain has been reported to be involved in variable biological functions including organogenesis, aging, infection, inflammation, biodefense, oncogenesis, cancer metastasis, tissue degeneration, regeneration and apoptosis. Glycomics is a glyco-engineering technology devoted to improve the therapeutic effect of conventional medicines by modifying its sugar chain so as to elevate both the activity and the stability, and also to minimize the effective dose and the adverse effects of them.

After the introduction in 1986 by Ortho Biotech Inc. of the first therapeutic antibody 'OKT3', a murine-derived antibody for the treatment of kidney transplant rejection, Centocor Inc. has developed 'Reopro' for inhibiting a platelet aggregation and 'Remicade' for treating Crohn's disease in 1994; and Genentech Inc., 'Herceptin' for treating breast cancer and 'Rituxan' for treating non-Nodgkins lymphoma (NHL). The development of the therapeutic antibody has been pursued in the fields of (i) technologies for the construction of chimeric, humanized and human antibodies (Jain, M. et al., Trend in Biotechnology, 25(7), 307-316, 2007), (ii) technologies for increased therapeutic effects of antibodies using Fc engineering (Lazar, G. A. et al., PNAS, 103(11), 4005-4010, 2006) or glycomics (Shuster, M. et al., Cancer Research, 65(17), 7934-7941, 2005; and Yamane-Ohnuki, N. et al., Biotechnology and Bioengineering, 87(5), 614-622, 2004), and (iii) technologies for enhanced efficiency in the production and purification of antibodies (Roque, A. C. et al., Biotechnology Progress, 20(3), 639-654, 2004).

The activity of a therapeutic antibody depends on its interaction with the Fc region receptor (FcrRIII) of natural killer (NK) cell, and it has been demonstrated that the removal of fucose from the sugar chain attached to Asn297 in Fc region of IgG leads to markedly strengthened interaction between the Fc region of IgG and Fc region receptor of NK cell, resulting in enhanced antibody-dependent cellular toxicity (ADCC) (Shields, R. L. et al., The Journal of Biological Chemistry, 277(30), 26733-26740, 2002).

Also, methods for elevating the ADCC of an antibody by reducing the fucose content in the sugar chain of the antibody have been disclosed, examples of which included methods for producing alpha 1,6-fucosyl transferase (FUT8) knock-out cell lines (Yamane-Ohnuki, N. et al., Biotechnology and Bioengineering, 87(5), 614-622, 2004; and U.S. Patent Publication No. 2004-0110704), methods for lowering the FUT8 expression using RNA interference (Mori, K. et al., Biotechnology and Bioengineering, 88(7), 901-908, 2004), and methods for inhibiting the expression of enzymes (e.g., GMD, Fx and GFPP) related to the synthesis of GDP-fucose which is a substrate for fucose synthesis (U.S. Patent Publication No. 2004-0093621).

Further, disclosed are method for reducing the fucose content by overexpressing N-acetylglycosaminyltrasnferase III (GnTIII) to inhibit the attachment of fucose to the sugar chain structure (Shuster, M. et al., Cancer Research, 65(17), 7934-7941, 2005, and U.S. Patent No. 6,602,684) and a method for preparing a therapeutic antibody having enhanced ADCC by replacing the $239^{th}$ serine with aspartic acid, the $332^{nd}$ isoleucine with glutamic acid and the $330^{th}$ alanine with leucine, respectively, of the antibody Fc region so as to increase the interaction with Fc region receptor (Lazar, G. A. et al., PNAS, 103(11), 4005-4010, 2006).

Fucosidase represents a set of enzymes that remove fucose from the sugar chain of protein and others including carbohydrates through hydrolysis, and it can be devided into tissue fucosidase (alpha-L-fucosidase-1, FUCA1) and plasma fucosidase (alpha-L-fucosidase-2, FUCA2) according to its localization. FUCA1 is a lysosomal protein known to have low pH-triggering activity, while FUCA2 is a cell membrane or soluble protein which is activated under a neutral condition (Aviles, M. et al., Biochemical journal, 318, 821-831, 1996). However, there has been no attempt to modify antibody-expressing host cells by way of manipulating the activity of fucosidase, e.g., to make fucosidase localized in the Golgi apparatus involved in antibody secretion, so that fucose in the sugar chain structure can be effectively removed.

FUT8 is a type II integral membrane protein localized in the Golgi apparatus, which has C-terminal catalytic domain located in the lumen and N-terminal cytoplasmic tail (CT) (Milland, J. et al., The Journal of Biological Chemistry, 277(12), 10374-10378, 2002). The CT, the transmembrane domain (TM), and the stem region (stem) of FUT8 have been suggested to play a significantly concerted role in the protein localization and retention in the Golgi apparatus, but the exact mechanism of action has not known, e.g., whether these enzymes form a dimer or not in action (Nilsson, T. et al., The EMBO Journal, 13(3), 562-574, 1994). Further, there has never been disclosed to date a method for inhibiting the enzyme activity using the CT, TM or stem region.

Accordingly, the present inventors have endeavored to develop an effective and new method for reducing the fucose content in an antibody, and have found that the fucose content of an antibody can be markedly reduced and the therapeutic effect of the antibody can be improved by a method comprising making fucosidase or its derivatives localized in the Golgi apparatus or expressing a recombinant protein using a FUT8 localization domain (a domain including CT, TM and stem region).

## SUMMARY OF THE INVENTION

Accordingly, it is an object of the present invention to provide a method for reducing the fucose content of a recombinant protein, which comprises expressing in an animal cell the recombinant protein and FUCA1, an FUCA1 mutant, FUCA2, or a fragment of FUT8 localization domain; or with a fusion protein of a fragment of FUT8 localization domain and a fragment of FUCA1, a FUCA1 mutant or FUCA2.

In accordance with one aspect of the present invention, there is provided a method for reducing the fucose content of a recombinant protein, which comprises expressing in an animal cell the recombinant protein and one or more proteins selected from the group consisting of: a) FUCA1 having the amino acid sequence of SEQ ID NO: 6; b) an FUCA1 mutant having an amino acid sequence obtained by replacing asparagine of the amino acid sequence of FUCA1 with other amino acid; c) FUCA2 having the amino acid sequence of SEQ ID NO: 7; and d) a fragment of the localization domain of FUT8 having the amino acid sequence of SEQ ID NO: 1.

In accordance with another aspect of the present invention, there is provided a method for reducing the fucose content of a recombinant protein, which comprises expressing in an animal cell the recombinant protein and a fusion protein obtained by fusing a fragment of the localization domain of FUT8 having the amino acid sequence of SEQ ID NO: 1 with a protein selected from the group consisting of: a) a fragment of FUCA1, which has the amino acid sequence obtained by deleting $1^{st}$ to $26^{th}$ amino acids of the amino acid sequence of SEQ ID NO: 6; b) a fragment of FUCA2, which has the amino acid sequence obtained by deleting $1^{st}$ to $28^{th}$ amino acids of the amino acid sequence of SEQ ID NO: 7; and c) a mutant of FUCA1 fragment, which has the amino acid sequence obtained by replacing asparagine of the amino acid sequence of the fragment of FUCA1 with other amino acid.

## BRIEF DESCRIPTION OF THE DRAWINGS

The above and other objects and features of the present invention will become apparent from the following description of the invention, when taken in conjunction with the accompanying drawings, which respectively show:

FIG. 1: The gene structures of the modifying proteins obtained using FUT8 and/or fucosidase for the modification of the sugar chain;
FIG. 2A: Western blot analysis of CHO cells expressing glyco-modifying gene of FUT8, FUCA1 or FUCA2 mutant;
FIG. 2B: RT-PCR analysis for subclones individually isolated from FUT8-stem1 CHO cells expressing glyco-modifying gene ofFUT8-steml;
FIGs. 3A and 3B: FACS analysis using LCA lectin showing fucose contents in CHO host cell lines containing sugar chain modifying genes;
FIG. 4: The fucose to four N-acetylglucosamine ratios determined by monosaccharides analysis using BIO-LC of antibodies produced in sugar chain-modified cell lines;
FIG. 5A: The analysis of complement-dependent cytotoxicitiy (CDC) of antibodies produced in sugar chain-modified cell lines;
FIG. 5B: The analysis of binding affinity of antibodies produced in sugar chain-modified cell lines with Fc gamma receptor IIIa (Fc RIIIa) expressed in CHO cell; and
FIG. 5C: The analysis of antibody-dependent cellular toxicity (ADCC) of antibodies produced in sugar chain-modified cell lines.

## DETAILED DESCRIPTION OF THE INVENTION

The present invention provides a method for reducing the fucose content of a recombinant protein, which comprises expressing in an animal cell the recombinant protein and one or more proteins selected from the group consisting of:

a) FUCA1 having the amino acid sequence of SEQ ID NO: 6;
b) an FUCA1 mutant having an amino acid sequence obtained by replacing asparagine of the amino acid sequence of FUCA1 with other amino acid;
c) FUCA2 having the amino acid sequence of SEQ ID NO: 7; and
d) a fragment of the localization domain of FUT8 having the amino acid sequence of SEQ ID NO: 1.

In the present invention, there is provided a method for reducing the fucose content of a therapeutic antibody obtained from a conventional antibody-producing animal cell line, which comprises the step of modifying the antibody-producing animal cell line by glyco-engineering using fucosidase hydrolyzing alpha-1,6-fucose, which binds to the asparagine residue of the N-acethyl glucosamine sugar chain structure of antibody Fc region, and/or alpha-1,6-fucosyltransferase involved in the synthesis of alpha-1,6-fucose.
In the present invention, the animal cell is first transfected with an expression vector for the recombinant protein and then one or more proteins selected from the group consisting of a) to d) are expressed therein.
In the method of the present invention, the "expression vector" for the recombinant protein can be introduced into an animal cell after overexpressing one or more proteins selected from the group consisting of a) to d) in the animal cell.
In the present invention, the procedure of expressing one or more of the proteins a) to d) is conducted by introducing into the animal cell i) a recombinant vector comprising a DNA encoding the proteins or ii) recombinant vectors each comprising a DNA encoding any one of the proteins a) to d).
In the present invention, the "recombinant protein" is preferably a glycoprotein having alpha-1,6-fucose on the N-acetylglucosamine reducing sugar terminal of the glycoprotein's carbohydrate moiety. Further, representative examples of the recombinant protein include antibodies, and the antibodies may be human IgGs such as IgG1, IgG2, IgG3 and IgG4; or chimeric, humanized or human antibodies.
In the present invention, the "animal cell" may be one of the known animal cells capable of producing a glycoprotein including an antibody, and representative examples of the "animal cell" include but not limited to the cells of CHO (Chinese hamster ovary), rat myeloma, BHK (Baby hamster kidney), hybridoma, Namalwa, embryonic stem and fertilized egg.
In the present invention, "FUCA1" " and "FUCA2" may be proteins having the amino acid sequences of SEQ ID NOs: 6 and 7, respectively.
In the present invention, the "FUCA1 mutant" may be a protein having an amino acid sequence obtained by replacing the 263$^{rd}$ asparagine of FUCA1 having the amino acid sequence of SEQ ID NO: 6 with other amino acid, e.g., valine.
In the present invention, the "fragment of FUT8 localization domain"(or "fragment of the localization domain of FUT8) may be a protein obtained by deleting the catalytic domain from wild type FUT8, which can reduce the content of fucose attached to antibody Fc regions by inhibiting the FUT8 retention in the Golgi apparatus or repressing FUT8 activity by inducing an inappropriate FUT8 aggregate, when overexpressed in a cell line expressing antibody. The fragment of FUT8 localization domain may comprise the cytoplasmic tail (CT), the transmembrane domain (TM) or the stem region (stem), which is known to be involved in the localization and retention of FUT8 in Golgi apparatus. Consequently, the fragment of FUT8 localization domain may be a protein having an amino acid sequence consisting of the 1 to n$^{th}$ amino acids of FUT8 having the amino acid sequence of SEQ ID NO: 1, wherein n is an integer ranging from 30 to 200. Preferred examples of the fragment of FUT8 localization domain include a fragment of SEQ ID NO: 2 wherein n is 30 (comprising CT and TM in FUT8), a fragment of SEQ ID NO: 3 wherein n is 101 (comprising CT, TM and a part of stem in FUT8), a fragment of SEQ ID NO: 4 wherein n is 125 (comprising CT, TM and a part of stem in FUT8; "stem 1") and a fragment of SEQ ID NO: 5 wherein n is 200 (comprising CT, TM and a part of stem in FUT8; "stem 2").
Further, the present invention provides a method for reducing the fucose content of a recombinant protein, which comprises expressing in an animal cell the recombinant protein and a fusion protein obtained by fusing a fragment of the localization domain of FUT8 having the amino acid sequence of SEQ ID NO: 1 with a protein selected from the group consisting of:

a) a fragment of FUCA1, which has the amino acid sequence obtained by deleting 1$^{st}$ to 26$^{th}$ amino acids of the amino acid sequence of SEQ ID NO: 6;
b) a fragment of FUCA2, which has the amino acid sequence obtained by deleting 1$^{st}$ to 28$^{th}$ amino acids of the amino acid sequence of SEQ ID NO: 7; and
c) a mutant of FUCA1 fragment, which has the amino acid sequence obtained by replacing asparagine of the amino acid sequence of the fragment of FUCA1 with other amino acid.

In the method of the present invention, the animal cell is first transfected with an expression vector for the recombinant protein and then one or more proteins selected from the group consisting of a) to c) are expressed therein.
In the method of the present invention, the "expression vector" for the recombinant protein may be introduced into the animal cell after expressing the fusion protein of the fragment of FUT8 localization domain and a fragment selected from the group consisting of a) to c) in the animal cell.

In the present invention, the "fragment of FUCA1" (or "FUCA1 fragment") and the "fragment of FUCA2" (or "FUCA2 fragment") may be proteins obtained by deleting signal sequences from FUCA1 and FUCA2 having the amino acid sequences of SEQ ID NOs: 6 and 7, respectively.

In the present invention, the "mutant of FUCA1 fragment" may be a protein having the amino acid sequence obtained by replacing asparagine of the fragment of FUCA1 with other amino acid, e.g., valine. In one embodiment of the present invention, the mutant of FUCA1 fragment may be a protein having the amino acid sequence obtained by replacing $263^{rd}$ asparagine with other amino acid, e.g., valine, and deleting $1^{st}$ to $26^{th}$ amino acids from the FUCA1 amino acid sequence of SEQ ID NO: 6. Such modification can make fucosidase lose its lysosome-targeting property so as to be localized to other intracellular regions such as Golgi apparatus.

In the present invention, the fusion protein may be a protein obtained by fusing the catalytic domain of FUCA1, FUCA2 or FUCA1 mutant without the signal sequence thereof with the fragment of FUT8 localization domain described above. Representative examples of the fusion protein include a fusion protein prepared by fusing a fragment of FUT8 localization domain having the amino acid sequence of SEQ ID NO: 3 with a FUCA1 fragment having the amino acid sequence obtained by deleting $1^{st}$ to $26^{th}$ amino acids from the amino acid sequence of SEQ ID NO: 6; a fusion protein prepared by fusing a fragment of FUT8 localization domain having the amino acid sequence of SEQ ID NO: 3 with a FUCA2 fragment having the amino acid sequence obtained by deleting $1^{st}$ to $28^{th}$ amino acids from the amino acid sequence of SEQ ID NO: 7; a fusion protein prepared by fusing a fragment of FUT8 localization domain having the amino acid sequence of SEQ ID NO: 2 with a mutant of FUCA1 fragment, which has the amino acid sequence obtained by replacing the $263^{rd}$ aspargine with valine and deleting $1^{st}$ to $26^{th}$ amino acids from the amino acid sequence of SEQ ID NO: 6; and a fusion protein prepared by fusing a fragment of FUT8 localization domain having the amino acid sequence of SEQ ID NO: 3 with a mutant of FUCA1 fragment, which has the amino acid sequence obtained by replacing the $263^{rd}$ aspargine with valine and deleting $1^{st}$ to $26^{th}$ amino acids from the amino acid sequence of SEQ ID NO: 6.

The recombinant protein, the animal cell and the fragment of FUT8 localization domain are the same as described above, respectively.

In the method of the present invention, the procedure of expressing the fusion protein is conducted by introducing into the animal cell i) a recombinant vector comprising a DNA encoding the fusion proteins or ii) recombinant vector each comprising a DNA encoding anyone of the fusion proteins.

Gene structures of FUT8 and the fragments of FUT8 localization domain; FUCA 1, FUCA2 and FUCA1 mutant; and the inventive fusion proteins, used for the modification of sugar chain in the present invention, are shown in FIG. 1.

In the present invention, the "recombinant protein" is preferably a glycoprotein having alpha-1,6-fucose in its structure, such as an antibody. Alpha-1,6-fucose has a feature of existing on the N-acetylglucosamine reducing sugar terminal of the glycoprotein's carbohydrate moiety. When the recombinant protein is an antibody, the antibody expressed according to the method of the present invention exhibits a reduced fucose content in its Fc region, which leads to the improvement in the therapeutic activity thereof.

The following Examples are intended to further illustrate the present invention without limiting its scope.

Example 1: Construction of expression vectors for FUCA1, FUCA2 and FUCA1 mutant

In order to construct expression vectors for FUCA1, FUCA2 and FUCA1 mutant, cDNAs encoding FUCA1, FUCA2 and FUCA1 mutant were each prepared by RT-PCR using human liver total RNA (Clontech, cat.636531, lot. 5070344) as a template, and cloned into pcDNA3.1 B(-)Myc-His vector (Invitrogen, INV-V855-20, US; hereinafter, referred to as "pcDNA") using restriction sites of NheI (NEB,131L)/XhoI(NEB,146L).

Specifically, a cDNA was synthesized from the human liver total RNA using Superscript first-strand synthesis kit (Invitrogen, 11904-018), and whole sequences encoding FUCA1 and FUCA2 were obtained by employing the synthesized cDNA as a template and primer pairs of SEQ ID NOs: 8 and 9 and SEQ ID NOs: 10 and 11, respectively. pcDNA (Invitrogen) and the whole sequences encoding FUCA1 or FUCA2 were digested with NheI and XhoI restriction enzymes, and the resulting DNAs were ligated to each other using DNA ligation kit (TAKARA, 6023). *E. coli* (DH5$\alpha$) cells were transformed with the resulting ligated DNAs and the vectors containing FUCA1 and FUCA2 DNAs were isolated and designated pcDNA-FUCA and pcDNA-FUCA2, respectively. The sequences of the vectors thus obtained were confirmed through sequence analysis.

FUCA1 mutant ("FUCA1NV") is a protein obtained by replacing the $263^{rd}$ amino acid, i.e., asparagine, of FUCA1 with valine. A cDNA encoding FUCK1 mutant was obtained by amplifying its 5' fragment and 3' fragment using primer pairs of SEQ ID NOs: 8 and 12 and SEQ ID NOs: 9 and 13, respectively, and performing overlap PCR using primers of SEQ ID NOs: 8 and 9. In the same manner as described above, the cDNA encoding FUCA1 mutant was inserted into pcDNA (Invitrogen) using NheI/XhoI restriction sites to obtain pcDNA-FUCA1NV

Plasmids designated pcDNA-FUCA1, pcDNA-FUCA2 and pcDNA-FUCA1NV were each harvested by using Endofree plasmid maxi kit (Quigen, 12362).

Example 2: Construction of expression vectors for fragments of FUT8 localization domain

A cDNA encoding FUT8 was prepared by RT-PCR using human liver total RNA as a template, and cloned in pcDNA vector (Invitrogen) using NheI/XhoI restriction sites.

Specifically, a cDNA was synthesized from the human liver total RNA using Superscript first-strand synthesis kit, and whole sequence encoding FUT8 was obtained by employing the synthesized cDNA as a template and primer pairs of SEQ ID NOs: 14 and 15. pcDNA (Invitrogen) and the whole sequence encoding FUT8 were digested with NheI and XhoI restriction enzymes, and the resulting DNAs were ligated to each other using DNA ligation kit. *E. coli* (DH5$\alpha$) cells were transformed with the resulting ligated DNAs and the vector containing FUT8 DNA were isolated and designated pcDNA-FUT8. The sequences of the vectors thus obtained were confirmed through sequence analysis, and the plasmid DNA was harvested by using Endofree plasmid maxi kit.

DNAs encoding fragments of FUT8 localization domain, which have amino acid sequences of 1$^{st}$ to 30$^{th}$ (CT/TM; SEQ ID NO: 2), 1$^{st}$ to 125$^{th}$ (stem1; SEQ ID NO: 4) and 1$^{st}$ to 200$^{th}$ (stem2; SEQ ID NO: 5) amino acids of FUT8, respectively, were amplified using pcDNA-FUT8 as a template and primer pairs of SEQ ID NOs: 14 and 16, SEQ ID NOs: 14 and 17 and SEQ ID NOs: 14 and 18, respectively. Then, in the same manner as described above, the amplified DNAs were each cloned in pcDNA to obtain pcDNA-FUT8-CT/TM, pcDNA-FUT8-stem1 and pcDNA-FUT8-stem2.

Example 3: Construction of expression vectors for fusion proteins between a fragment of FUT8 localization domain and the catalytic domain of FUCA1, FUCA2 or FUCA1 mutant

DNAs encoding fusion proteins between a fragment of FUT8 localization domain and the catalytic domain of FUCA 1, FUCA2 or FUCA1 mutant (without the signal sequence of FUCA1, FUCA2 or FUCA1 mutant) were prepared by performing overlap PCR using a DNA encoding a fragment of FUT8 localization domain and a DNA encoding a fragment of FUCA1, or FUCA2 or FUCA1 mutant, respectively.

Specifically, 5' fragment of a DNA encoding a fusion protein between a fragment of FUT8 localization domain having the amino acid sequence of SEQ ID NO: 3 (i.e., 1$^{st}$ to 101$^{st}$ amino acids of FUT8) and a fragment of FUCA1 (i.e., a fragment consisting of 27$^{th}$ to 461$^{st}$ amino acids of FUCA1) (hereinafter, the fusion protein is referred to as "FLD-FUCA1") was amplified by PCR using pcDNA-FUT8 as a template and primers of SEQ ID NOs: 14 and 19, and 3' fragment of the DNA encoding FLD-FUCA1 was amplified by employing pcDNA-FUCA1 as a template and primers of SEQ ID NOs: 9 and 20. Overlap PCR was performed using the resulting DNA fragments and primers of SEQ ID NOs: 14 and 9 to obtain a DNA encoding FLD-FUCA1. The thus obtained DNA was cloned in pcDNA using NheI/XhoI restriction sites to obtain pcDNA-FLD-FUCA1.

Meanwhile, 5' fragment of a DNA encoding a fusion protein between a fragment of FUT8 localization domain having the amino acid sequence of SEQ ID NO: 3 (i.e., 1$^{st}$ to 101$^{st}$ amino acids of FUT8) and a fragment of FUCA2 (i.e., a fragment consisting of 29$^{th}$ to 467$^{th}$ amino acids of FUCA2) (hereinafter, the fusion protein is referred to as "FLD-FUCA2") was amplified by PCR using pcDNA-FUT8 as a template and primers of SEQ ID NOs: 14 and 21, and 3' fragment of the DNA encoding FLD-FUCA2 was amplified by employing pcDNA-FUCA2 as a template and primers of SEQ ID NOs: 11 and 22. Overlap PCR was performed using the resulting DNA fragments and primers of SEQ ID NOs: 14 and 11 to obtain a DNA encoding FLD-FUCA2. The thus obtained DNA was cloned in pcDNA using NheI/XhoI restriction sites to obtain pcDNA-FLD-FUCA2.

Further, 5' fragment of a DNA encoding a fusion protein between a fragment of FUT8 localization domain having the amino acid sequence of SEQ ID NO: 2 (i.e., 1$^{st}$ to 30$^{th}$ amino acids of FUT8) and a fragment of FUCA1 mutant

(i.e., a fragment consisting of 27$^{th}$ to 461$^{st}$ amino acids of FUCA1 mutant) (hereinafter, the fusion protein is referred to as "FLD1-FUCA1NV") was amplified by PCR using pcDNA-FUT8 as a template and primers of SEQ ID NOs: 14 and 23, and 3' fragment of the DNA encoding FLD1-FUCA1NV was amplified by employing pcDNA-FUCA1NV as a template and primers of SEQ ID NOs: 9 and 24; and 5' fragment of a DNA encoding a fusion protein between a fragment of FUT8 localization domain having the amino acid sequence of SEQ ID NO: 3 (i.e., 1$^{st}$ to 101$^{st}$ amino acids of FUT8) and a fragment of FUCA1 mutant (i.e., a fragment consisting of 27$^{th}$ to 461$^{st}$ amino acids of FUCA1 mutant) (hereinafter, the fusion protein is referred to as "FLD2-FUCA1NV") was amplified by PCR using pcDNA-FUT8 as a template and primers of SEQ ID NOs: 14 and 19, and 3' fragment of the DNA encoding FLD2-FUCA1NV was amplified by employing pcDNA-FUCA1NV as a template and primers of SEQ ID NOs: 9 and 20. Then, overlap PCR was performed using the resulting DNA fragments and primers of SEQ ID NOs: 14 and 9 to obtain DNAs encoding FLD1-FUCA1NV and FLD2-FUCA1NV, which were cloned in pcDNA using NheI/XhoI restriction sites to obtain pcDNA-FLD1-FUCA1NV and pcDNA-FLD2-FUCA1NV, respectively.

The thus obtained plasmids designated pcDNA-FLD-FUCA1, pcDNA-FLD-FUCA2, pcDNA-FLD1-FUCA1NV and pcDNA-FLD2-FUCA1NV were each harvested by using Endofree plasmid maxi kit (Quigen, 12362).

Example 4: Preparation of control antibody-expressing cell line using siRNA

A cell line expressing control antibody exhibiting a reduced fucose content was prepared by introducing FUT8 siRNA into a cell line expressing a therapeutic antibody.
Specifically, a DNA encoding a control antibody, i.e., Rituxan, which is an anti-CD20 therapeutic antibody (U.S. Patent No. 5,736,137) was inserted into pMSG vector (Korean Patent No. 408844; KCCM 10202), and CHO-DG44 (dhfr-) cells (Dr. Lawrence Chasin, Columbia University, New York, USA) were transfected with the resulting plasmid and pDCH1P plasmid (Dr. Lawrence Chasin, Columbia University, New York, USA) expressing dihydrofolate reductase (DHFR). After harvesting the transfected colonies, the cells were adapted to gradually increasing concentrations (until 1 μM) of methotrexate (MTX), which is a folate analog, to obtain CHO-Rituxan cell line highly expressing Rituxan.
A cell line expressing control antibody exhibiting a reduced fucose content in its Fc region was prepared as follows, by employing two conventional siRNA sequences (Mori, K. et al., Biotechnology and Bioengineering, 88(7), 901-908,2004).
In order to prepare B form (FUT8 siB) known to inhibit the region of SEQ ID NO: 25 in the whole nucleotide sequence of FUT8, primers of SEQ ID NOs: 26 and 27 were ligated to each other; and in order to prepare R form (FUT8 siR) known to inhibit the region of SEQ ID NO: 28 in the whole nucleotide sequence of FUT8, primers of SEQ ID NOs: 29 and 30 were ligated to each other. The ligated primers were each cloned in pSilencer 2.1-U6 hygro vector (Ambion, 5760) and transfected into CHO-Rituxan cells in the same manner as described above. The transfected cells were subjected to a primary selection using hygromycin as a selection marker, followed by final selection through FACS (fluorescence activated cell sorting) analysis using LCA (biotilated-lens culinaris agglutinin; Vector Lab, B-1045(SO925)) and phycoerythrin (PE) streptavidin (Vector Lab, SA-5007(R1209)) to obtain CHO-R-siRNA cell lines.

Example 5: Preparation of cell lines expressin therapeutic antibody with modified sugar chain

For an example, CHO-Rituxan cells expressing the antibody of Rituxan were transfected with the plasmids comprising sugar chain modifying genes, pcDNA-FUT8, pcDNA-FUT8-CT/TM, pcDNA-FUT8-steml, pcDNA-FUT8-stem2, pcDNA-FUCA1, pcDNA-FUCA2, pcDNA-FUCA1NV, pcDNA-FLD-FUCA1, pcDNA-FLD-FUCA2, pcDNA-FLD1-FUCA1NV and pcDNA-FLD2-FUCA1NV, obtained in Examples 1 to 3 by using lipofectamine 2000 (Invitrogen, 11668-019 (1369361)), and the recombinant cell lines were each selected using neomycin as a selection marker. The cell lines thus obtained were designated CHO-R-FUT8, CHO-R-FUTB-CT/TM, CHO-R-FUT8-stem1, CHO-R-FUT8-stem2, CHO-R-FUCA1, CHO-R-FUCA2, CHO-R-FUCA1NV, CHO-R-FLD-FUCA1, CHO-R-FLD-FUCA2, CHO-R-FLD1-FUCA1NV and CHO-R-FLD2-FUCA1NV, respectively.
In order to select CHO cell lines highly expressing sugar chain modifying genes, the cell lines thus obtained were each subjected to western blot analysis using rabbit anti-His antibody (SantaCruz, His-probe (G-18) rabbit polyclonal, sc804 (H3006)) and Goat anti-rabbit antibody (KPL, Goat anti-rabbit IgG(H+L)-HRP, 074-1506), and to RT-PCR analysis, and the results are shown in FIGs. 2A and 2B.
As shown in FIG. 2A, the sugar chain modifying gene of FUT8, FUCA1 or FUCA2 mutant was over-expressed in the CHO cell lines. Further, as shown in FIG. 2B, the sugar chain modifying gene of FUT8-steml was over-expressed in the individually isolated subclones of the FUT8-stem1 CHO cell lines. The lane mix is transfectants comprising mixed isolated subclones of FUT8-stem1; the lane CHO is CHO DG44 cell line; and mock is a transfectant prepared using only vector.
In addition, the results from FACS analysis using LCA (Vector Lab, B-1045(S0925)) and phycoerythrin streptavidin (Vector Lab, SA-5007(R1209)) are shown in FIGs. 3A and 3B.
As shown in FIG. 3A, the inventive cell lines exhibited low affmities to LCA, as compared with CHO-Rituxan cells lines. Further, as shown in FIG. 3B, the relative FACS mean values of the inventive cell lines were reduced by about 10~35% from that of CHO-Rituxan cells, the values being, CHO-R-siRNA: 0.17, CHO-R-FUCA1: 0.93, CHO-R-FUCA2: 0.79, CHO-R-FUT8: 0.87, CHO-R-FUCA1NV: 0.65, CHO-R-FLD-FUCA2: 0.74, CHO-R-FLD1FUCA1NV: 0.79, CHO-R-FLD2-FUCA1NV: 0.80, CHO-R-FUT8-CT/TM: 0.80, CHO-R-FUT8-stem1: 0.79 and CHO-R-stem2: 0.73.
These results suggest that FUT8 localization domain, FUCA1, FUCA2, FUCA1 mutant, and a fusion protein of a fragment of FUT8 localization domain and a fragment of FUCA1, FUCA2 or FUCA1 mutant can be advantageously used for reducing fucose contents in sugar chain structures of glycoproteins.

Example 6: Analysis for sugar chains of antibodies

Antibodies produced by the cell lines prepared in Example 5, which expresses therapeutic antibody with modified sugar chain, were purified and subjected to a monosaccharide analysis using BIO-LC system (DC ICS 3000 system, DIONEX, 06110276) to quantitatively analyze the sugar chains in the Fc regions thereof as follows.
In embodiment, each cultured medium of the cell lines was purified using a Protein G Sepharose (Amersham, 17-0618-01) column. In this purification, 20 mM sodium phosphate buffer (pH 7.0) was employed as a binding buffer; 0.5 M glycine (pH 2.7), as a diluting buffer; and 1 M Tris-HCl (pH 9.0), as a neutralizing buffer. The purity of each purified antibody was analyzed by silver staining.

Each purified antibody was heated at 100°C for 4 hrs in 4 M TFA (Trifluoroacetic acid) to separate monosaccharides therefrom, and dried in a vacuum dryer. The resulting residues were dissolved in deionized water and analyzed using BIO-LC system to determine fucose contents. The BIO-LC system was equipped with ED detector (DIONEX, 06110046), amino trap column (DIONEX, 046122) as a guard column and CarboPac PA 10 column (DIONEX, 046110) as an analysis column.

Each sample of 25μl was used in analyzing the monosaccharides, and DI water and 200 mM NaOH (Fisher, SS254-1) were employed as eluents. The analysis conditions and the time-dependent changes in the concentration of the eluents are shown in Tables 1 and 2, respectively.

Table 1

| Analysis conditions | |
|---|---|
| Amount of sample | 25 μl |
| Analysis column | CarboPac PA 10 (4x250mm) |
| Guard column | Amino-trap column (4x50mm) or PA 10 |
| Detector | ED detector |
| Eluent A | Deionized water |
| Eluent B | 200 mM NaOH |

Table 2

| Time (min) | Concentration of eluent A (%) | Concentration of eluent B (%) | Condition |
|---|---|---|---|
| 0 | 91 | 9 | 18mM NaOH |
| 20 | 91 | 9 | 18mM NaOH |
| 20.1 | 0 | 100 | Washing condition |
| 25 | 0 | 100 | Washing condition |
| 25.1 | 91 | 9 | Re-equilibration condition |
| 30 | 91 | 9 | Re-equilibration condition |

The content of monosaccarides was represented as ratios of fucose to four N-acetylglucosamines existed in the frame of sugar chain of antibody Fc region. As shown in FIG. 4, the inventive cell lines exhibited reduced ratio of fucose/N-acetylglucosamine by about 10-25% as compared with that of CHO-Rituxan cell lines, the ratios being, CHO-Rituxan: 1.01, CHO-R-siRNA: 0.10, CHO-R-FUCA1: 0.88, CHO-R-FUCA2: 0.90, CHO-R-FUT8: 1.03, CHO-R-FUCA1NV: 0.75, CHO-R-FLD-FUCA1: 0.90, CHO-R-FLD-FUCA2: 0.82, CHO-R-FLD1-FUCA1NV: 0.79, CHO-R-FLD2-FUCA1NV: 0.75, CHO-R-CT/TM: 0.78, CHO-R-FUT8-steml: 0.84 and CHO-R-FUT8-stem2: 0.76.

Similarly, the fragments of FUT8 localization domain reduced the fucose contents in antibody Fc regions by about 15 to 25% when introduced into therapeutic antibody-expressing cell lines. Further, the FUCA1 or FUCA2-overexpressing cell lines of the present invention exhibited reduced fucose contents by about 10%, and the inventive cell lines overexpressing a FUCA1 mutant, i.e., FUCA1NV, exhibited reduced fucose contents by about 25%. Furthermore, the inventive cell lines expressing fusion proteins of a fragment of FUT8 localization domain and a catalytic domain of FUCA1, FUCA2 or FUCA1 mutant exhibited reduced fucose contents by 10~25%.

These results suggest that the inventive antibody-expressing cell lines overexpressing fragments of FUT8 localization domain, FUCA1, FUCA2, FUCA1 mutant, and fusion proteins of a fragment of FUT8 and a fragment of FUCA1, FUCA2 or FUCA1 mutant can be advantageously used for producing therapeutic antibodies having low fucose contents.

Example 7: Analysis for the therapeutic effects of the antibodies with a modified sugar chain

The therapeutic effects of the inventive antibodies having modified sugar chain were analyzed by determining the complement-dependent cytotoxicities (CDC), the binding affinity with Fc region receptor and the antibody-dependent cellular cytotoxicities (ADCC) thereof.

Specifically, for the CDC test, CD20-expressing Daudi (ATCC CCL-213) B-cell lymphoma cells were treated with anti-CD20 antibodies purified from CHO-Rituxan, CHO-R-siRNA, CHO-R-FUT8-LD (CHO-R-FUT8-CT/TM and CHO-R-FUT8-stem1) and CHO-R-FUCA1NV cells, and standard human plasma (DADE Behring), respectively, and then, treated with WST-1 reagent (Roche), followed by determining the absorbance intensities of the cells at 450 nm and 690 nm (Hodoniczky J. et al., Biotechnology Progress, 21(6), 1644-1652, 2005). As shown in FIG. 5A, the antibodies purified from the inventive cell lines exhibited similar CDC values.

For measuring binding capacity of the antibody Fc region with an Fc receptor, an antibody Fc region receptor (FcrRIIIa) was obtained from human peripheral blood mononuclear cell (PBMC) using RT-PCR (Clémenceau, B. et. al., Blood, 107(12), 4669-4677, 2006). Specifically, in order to clone a gene encoding soluble antibody Fc region receptor (FcrRIIIa) (GenBank, X52645), total RNA was isolated from human PBMC, and subjected to RT-PCR using cDNA synthesis kit (Invitrogen, 18080-051) to obtain cDNA. PCR was performed using the obtained cDNA as a template and primers of SEQ ID NOs: 31 and 32 to obtain a DNA encoding FcrRIIIa-His, wherein the cytoplasmic tail domain and transmembrane domain were deleted from FcrRIIIa and 6 histidine (His) residues were attached to the extracellular domain following signal sequence at the 5' end thereof. FcrRIIIa-His DNA was cloned in pMSG vector to obtain a plasmid designated pMSG-FcrRIIIa-his. Plasmid pMSG-FcrRIIIa-his was transfected into CHO cells using lipofectamine 2000 (Invitrogen, 11668-019), and the expression of FcrRIIIa was amplified through MTX system. The finally selected CHO-FcrRIIIa-his cell line was incubated in a conventional cell culture medium, and 200 ml of the incubated medium was subjected to His column (Novagen) purification to obtain purified FcrRIIIa-His. The binding capacity of antibody Fc region with the Fc receptor was measured by ELISA using anti-His monoclonal antibody (QIAGEN, anti-4X his antibody) and antihuman F(ab)2-HRP (Jackson Lab, 309-036-006).

As shown in FIG. 5B, the absorbance values of the test cell lines were increased in an antibody dose-dependent manner, the inventive cell lines, and Rituxan having modified sugar chain exhibited higher binding affinity to FcrRIIIa-His by about 4 folds as compared with normal Rituxan.

For the ADCC (Antibody-dependent cellular toxicity) test, Daudi (ATCC CCL-213) cells ($1\times10^4$ cells/well) expressing CD20 were treated with Cr-51 to introduce Cr-51 thereinto, and then treated with purified antibodies produced from CHO-Rituxan, CHO-R-siRNA, CHO-R-FUT8-CT/TM, CHO-R-FUT8-stem1 and CHO-R-FUCA1NV cell lines and PBMC ($4\times10^5$ cells) isolated from the bloods of healthy volunteers, followed by measuring the amount of Cr-51 released from dead cells using a gamma counter. The ADCC of each antibody was calculated from the measured values using the following mathematical formula (Shinkawa, T. et al., Journal of Biological Chemistry, 278(5), 3466-3473, 2003):

$$\text{ADCC}(\%) = 100\times (\text{E-ST})/(\text{M-ST})$$

E: experimental release
ST: spontaneous release
M: maximum release

As shown in FIG. 5C, the antibodies having modified sugar chains produced from the inventive cell lines exhibited ADCC values increased by about 2 to 5 folds as compared with that of antibody having normal sugar chains.

While the invention has been described with respect to the above specific embodiments, it should be recognized that various modifications and changes may be made to the invention by those skilled in the art which also fall within the scope of the invention as defined by the appended claims.

## Claims

1. A method for reducing the fucose content of a recombinant protein, which comprises expressing in an animal cell the recombinant protein and one or more proteins selected from the group consisting of:

   a) FUCA1 having the amino acid sequence of SEQ ID NO: 6;
   b) an FUCA1 mutant having an amino acid sequence obtained by replacing asparagine of the amino acid sequence of FUCA1 with other amino acid;
   c) FUCA2 having the amino acid sequence of SEQ ID NO: 7; and
   d) a fragment of the localization domain of FUT8 having the amino acid sequence of SEQ ID NO: 1.

2. The method of claim 1, wherein the recombinant protein is a glycoprotein having alpha-1,6-fucose.

3. The method of claim 2, wherein the alpha-1,6-fucose exists on the N-acetylglucosamine reducing sugar terminal of the glycoprotein's carbohydrate moiety.

4. The method of claim 1, wherein the recombinant protein is an antibody.

5. The method of claim 4, wherein the fucose content of the antibody is reduced to elevate the therapeutic effect of the antibody.

6. The method of claim 1, wherein the animal cell is first transfected with an expression vector for the recombinant protein and then one or more proteins selected from the group consisting of a) to d) are expressed therein.

7. The method of claim 1, wherein an expression vector for the recombinant protein is introduced into the animal cell after expressing one or more proteins selected from the group consisting of a) to d) in the animal cell.

8. The method of claim 1, wherein the fragment of FUT8 localization domain has an amino acid sequence composed of the $1^{st}$ to $n^{th}$ amino acids of FUT8 having the amino acid sequence of SEQ ID NO: 1, n being an integer ranging from 30 to 200.

9. The method of claim 8, wherein n is 30 and the fragment of FUT8 localization domain has the amino acid sequence of SEQ ID NO: 2.

10. The method of claim 8, wherein n is 125 and the fragment of FUT8 localization domain has the amino acid sequence of SEQ ID NO: 4.

11. The method of claim 8, wherein n is 200 and the fragment of FUT8 localization domain has the amino acid sequence of SEQ ID NO: 5.

12. The method of claim 1, wherein the FUCA1 mutant has the amino acid sequence obtained by replacing the $263^{rd}$ asparagine of FUCA1 having the amino acid sequence of SEQ ID NO: 6 with valine.

13. The method of claim 1, wherein the animal cell is selected from the group consisting of cells of CHO (Chinese hamster ovary), rat myeloma, BHK (baby hamster kidney), hybridoma, Namalwa, embryonic stem and fertilized egg.

**14.** The method of claim 1, wherein the procedure of expressing one or more of the proteins a) to d) is conducted by introducing into the animal cell i) a recombinant vector comprising a DNA encoding the proteins or recombinant vectors each comprising a DNA encoding any one of the proteins a) to d).

**15.** A method for reducing the fucose content of a recombinant protein, which comprises expressing in an animal cell the recombinant protein and a fusion protein obtained by fusing a fragment of the localization domain of FUT8 having the amino acid sequence of SEQ ID NO: 1 with a protein selected from the group consisting of:

a) a fragment of FUCA1, which has the amino acid sequence obtained by deleting 1$^{st}$ to 26$^{th}$ amino acids of the amino acid sequence of SEQ ID NO: 6;
b) a fragment of FUCA2, which has the amino acid sequence obtained by deleting 1$^{st}$ to 28$^{th}$ amino acids of the amino acid sequence of SEQ ID NO: 7; and
c) a mutant of FUCA1 fragment, which has the amino acid sequence obtained by replacing asparagine of the amino acid sequence of the fragment of FUCA1 with other amino acid.

**16.** The method of claim 15, wherein the recombinant protein is a glycoprotein having alpha-1,6-fucose.

**17.** The method of claim 16, wherein the alpha-1,6-fucose exists on the N-acetylglucosamine reducing sugar terminal of the glycoprotein's carbohydrate moiety.

**18.** The method of claim 15, wherein the recombinant protein is an antibody.

**19.** The method of claim 18, wherein the fucose content of the antibody is reduced to elevate the therapeutic effect of the antibody.

**20.** The method of claim 15, wherein the animal cell is first transfected with an expression vector for the recombinant protein and then one or more proteins selected from the group consisting of a) to c) are expressed therein.

**21.** The method of claim 15, wherein an expression vector for the recombinant protein is introduced into then animal cell after expressing the fusion protein of the fragment of FUT8 localization domain and a fragment selected from the group consisting of a) to c) in the animal cell.

**22.** The method of claim 15, wherein the fragment of FUT8 localization domain has an amino sequence composed of 1$^{st}$ to nth amino acids of FUT8 having the amino acid sequence of SEQ ID NO: 1, n being an integer ranging from 30 to 200.

**23.** The method of claim 22, wherein n is 30 and the fragment of FUT8 localization domain has the amino acid sequence of SEQ ID NO: 2.

**24.** The method of claim 22, wherein n is 101 and the fragment of FUT8 localization domain has the amino acid sequence of SEQ ID NO: 3.

**25.** The method of claim 22, wherein n is 125 and the fragment of FUT8 localization domain has the amino acid sequence of SEQ ID NO: 4.

**26.** The method of claim 22, wherein n is 200 and the fragment of FUT8 localization domain has the amino acid sequence of SEQ ID NO: 5.

**27.** The method of claim 15, wherein the mutant of FUCA fragment has the amino acid sequence obtained by replacing the 263$^{rd}$ asparagine with valine and deleting 1$^{st}$ to 26$^{th}$ amino acids from the amino acid sequence of SEQ ID NO: 6.

**28.** The method of claim 15, wherein the fusion protein is a protein prepared by fusing a fragment of FUT8 localization domain having the amino acid sequence of SEQ ID NO: 3 with a fragment of FUCA1 having the amino acid sequence obtained by deleting 1$^{st}$ to 26$^{th}$ amino acids from the amino acid sequence of SEQ ID NO: 6.

**29.** The method of claim 15, wherein the fusion protein is a protein prepared by fusing a fragment of FUT8 localization domain having the amino acid sequence of SEQ ID NO: 3 with a fragment of FUCA2 having the amino acid sequence obtained by deleting 1st to 28th amino acids from the amino acid sequence of SEQ ID NO: 7.

**30.** The method of claim 15, wherein the fusion protein is a protein prepared by fusing a fragment of FUT8 localization domain having the amino acid sequence of SEQ ID NO: 2 with a mutant of FUCA1 fragment having the amino acid sequence obtained by replacing the 263rd asparagine with valine and deleting 1st to 26th amino acids from the amino acid sequence of SEQ ID NO: 6.

**31.** The method of claim 15, wherein the fusion protein is a protein prepared by fusing a fragment of FUT8 localization domain having the amino acid sequence of SEQ ID NO: 3 with a mutant of FUCA1 fragment having the amino acid sequence obtained by replacing the 263rd asparagine with valine and deleting 1st to 26th amino acids from the amino acid sequence of SEQ ID NO: 6.

**32.** The method of claim 15, wherein the animal cell is selected from the group consisting of cells of CHO (Chinese hamster ovary), rat myeloma, BHK (baby hamster kidney), hybridoma, Namalwa, embryonic stem and fertilized egg.

**33.** The method of claim 15, wherein the procedure of expressing the fusion protein is conducted by introducing into the animal cell i) a recombinant vector comprising a DNA encoding the fusion proteins or ii) recombinant vector each comprising a DNA encoding anyone of the fusion proteins.

**Amended claims in accordance with Rule 137(2) EPC.**

**1.** A method for reducing the fucose content of a recombinant protein, which comprises expressing in an animal cell the recombinant protein and one or more proteins selected from the group consisting of:

a) an FUCA mutant having an amino acid sequence obtained by replacing asparagine of FUCA1 having the amino acid sequence of SEQ ID NO: 6 with other amino acid; and
b) a fragment of the localization domain of FUT8 having the amino acid sequence of SEQ ID NO: 1.

**2.** The method of claim 1, wherein the recombinant protein is a glycoprotein having alpha-1,6-fucose.

**3.** The method of claim 2, wherein the alpha-1,6-fucose exists on the N-acetylglucosamine reducing sugar terminal of the glycoprotein's carbohydrate moiety.

**4.** The method of claim 1, wherein the recombinant protein is an antibody.

**5.** The method of claim 4, wherein the fucose content of the antibody is reduced to elevate the therapeutic effect of the antibody.

**6.** The method of claim 1, wherein the animal cell is first transfected with an expression vector for the recombinant protein and then one or more proteins selected from the group consisting of a) to b) are expressed therein.

**7.** The method of claim 1, wherein an expression vector for the recombinant protein is introduced into the animal cell after expressing one or more proteins selected from the group consisting of a) to b) in the animal cell.

**8.** The method of claim 1, wherein the fragment of FUT8 localization domain has an amino acid sequence composed of the 1st to nth amino acids of FUT8 having the amino acid sequence of SEQ ID NO: 1, n being an integer ranging from 30 to 200.

**9.** The method of claim 8, wherein n is 30 and the fragment of FUT8 localization domain has the amino acid sequence of SEQ ID NO: 2.

**10.** The method of claim 8, wherein n is 125 and the fragment of FUT8 localization domain has the amino acid sequence of SEQ ID NO: 4.

**11.** The method of claim 8, wherein n is 200 and the fragment of FUT8 localization domain has the amino acid sequence of SEQ ID NO: 5.

**12.** The method of claim 1, wherein the FUCA1 mutant has the amino acid sequence obtained by replacing the 263$^{rd}$ asparagine of FUCA1 having the amino acid sequence of SEQ ID NO: 6 with valine.

**13.** The method of claim 1, wherein the animal cell is selected from the group consisting of cells of CHO (Chinese hamster ovary), rat myeloma, BHK (baby hamster kidney), hybridoma, Namalwa, embryonic stem and fertilized egg.

**14.** The method of claim 1, wherein the procedure of expressing one or more of the proteins a) to b) is conducted by introducing into the animal cell i) a recombinant vector comprising a DNA encoding the proteins or ii) recombinant vectors each comprising a DNA encoding any one of the proteins a) to b).

**15.** A method for reducing the fucose content of a recombinant protein, which comprises expressing in an animal cell the recombinant protein and a fusion protein obtained by fusing a fragment of the localization domain of FUT8 having the amino acid sequence of SEQ ID NO: 1 with a protein selected from the group consisting of:

a) a fragment of FUCA1, which has the amino acid sequence obtained by deleting 1$^{st}$ to 26$^{th}$ amino acids of the amino acid sequence of SEQ ID NO: 6;
b) a fragment of FUCA2, which has the amino acid sequence obtained by deleting 1$^{st}$ to 28$^{th}$ amino acids of the amino acid sequence of SEQ ID NO: 7; and
c) a mutant of FUCA1 fragment, which has the amino acid sequence obtained by replacing asparagine of the amino acid sequence of the fragment of FUCA1 with other amino acid.

**16.** The method of claim 15, wherein the recombinant protein is a glycoprotein having alpha-1,6-fucose.

**17.** The method of claim 16, wherein the alpha-1,6-fucose exists on the N-acetylglucosamine reducing sugar terminal of the glycoprotein's carbohydrate moiety.

**18.** The method of claim 15, wherein the recombinant protein is an antibody.

**19.** The method of claim 18, wherein the fucose content of the antibody is reduced to elevate the therapeutic effect of the antibody.

**20.** The method of claim 15, wherein the animal cell is first transfected with an expression vector for the recombinant protein and then one or more proteins selected from the group consisting of a) to c) are expressed therein.

**21.** The method of claim 15, wherein an expression vector for the recombinant protein is introduced into then animal cell after expressing the fusion protein of the fragment of FUT8 localization domain and a fragment selected from the group consisting of a) to c) in the animal cell.

**22.** The method of claim 15, wherein the fragment of FUT8 localization domain has an amino sequence composed of 1$^{st}$ to n$^{th}$ amino acids of FUT8 having the amino acid sequence of SEQ ID NO: 1, n being an integer ranging from 30 to 200.

**23.** The method of claim 22, wherein n is 30 and the fragment of FUT8 localization domain has the amino acid sequence of SEQ ID NO: 2.

**24.** The method of claim 22, wherein n is 101 and the fragment of FUT8 localization domain has the amino acid sequence of SEQ ID NO: 3.

**25.** The method of claim 22, wherein n is 125 and the fragment of FUT8 localization domain has the amino acid sequence of SEQ ID NO: 4.

**26.** The method of claim 22, wherein n is 200 and the fragment of FUT8 localization domain has the amino acid sequence of SEQ ID NO: 5.

**27.** The method of claim 15, wherein the mutant of FUCA1 fragment has the amino acid sequence obtained by replacing the 263$^{rd}$ asparagine with valine and deleting 1$^{st}$ to 26$^{th}$ amino acids from the amino acid sequence of SEQ ID NO: 6.

**28.** The method of claim 15, wherein the fusion protein is a protein prepared by fusing a fragment of FUT8 localization domain having the amino acid sequence of SEQ ID NO: 3 with a fragment of FUCA1 having the amino acid sequence obtained by deleting $1^{st}$ to $26^{th}$ amino acids from the amino acid sequence of SEQ ID NO: 6.

**29.** The method of claim 15, wherein the fusion protein is a protein prepared by fusing a fragment of FUT8 localization domain having the amino acid sequence of SEQ ID NO: 3 with a fragment of FUCA2 having the amino acid sequence obtained by deleting $1^{st}$ to $28^{th}$ amino acids from the amino acid sequence of SEQ ID NO: 7.

**30.** The method of claim 15, wherein the fusion protein is a protein prepared by fusing a fragment of FUT8 localization domain having the amino acid sequence of SEQ ID NO: 2 with a mutant of FUCA1 fragment having the amino acid sequence obtained by replacing the $263^{rd}$ asparagine with valine and deleting $1^{st}$ to $26^{th}$ amino acids from the amino acid sequence of SEQ ID NO: 6.

**31.** The method of claim 15, wherein the fusion protein is a protein prepared by fusing a fragment of FUT8 localization domain having the amino acid sequence of SEQ ID NO: 3 with a mutant of FUCA1 fragment having the amino acid sequence obtained by replacing the $263^{rd}$ asparagine with valine and deleting $1^{st}$ to $26^{th}$ amino acids from the amino acid sequence of SEQ ID NO: 6.

**32.** The method of claim 15, wherein the animal cell is selected from the group consisting of cells of CHO (Chinese hamster ovary), rat myeloma, BHK (baby hamster kidney), hybridoma, Namalwa, embryonic stem and fertilized egg.

**33.** The method of claim 15, wherein the procedure of expressing the fusion protein is conducted by introducing into the animal cell i) a recombinant vector comprising a DNA encoding the fusion proteins or ii) recombinant vector each comprising a DNA encoding anyone of the fusion proteins.

FIG. 1

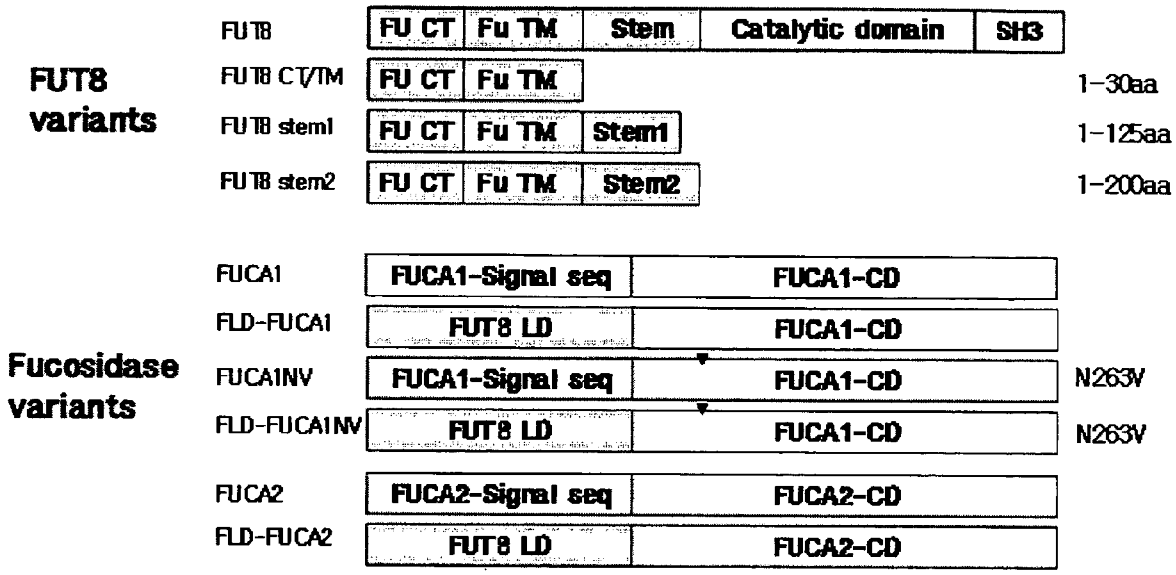
FUT8 variants
FUT8
FU CT
Fu TM
Stem
Catalytic domain
SH3
FUT8 CT/TM
1-30aa
FUT8 stem1
Stem1
1-125aa
FUT8 stem2
Stem2
1-200aa
Fucosidase variants
FUCA1
FUCA1-Signal seq
FUCA1-CD
FLD-FUCA1
FUT8 LD
FUCA1NV
N263V
FLD-FUCA1NV
N263V
FUCA2
FUCA2-Signal seq
FUCA2-CD
FLD-FUCA2

FIG. 2a

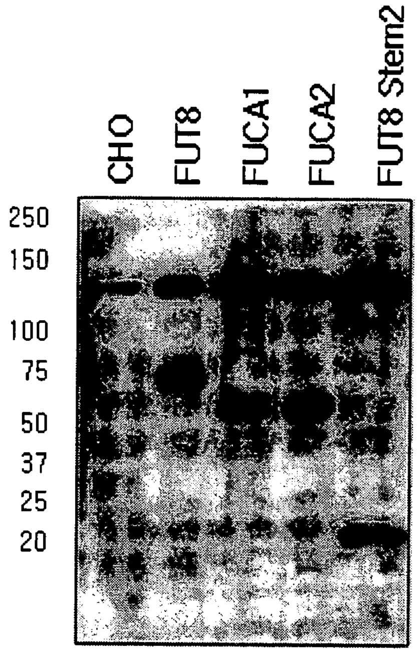
CHO
FUT8
FUCA1
FUCA2
FUT8 Stem2
250
150
100
75
50
37
25
20

FIG. 2b

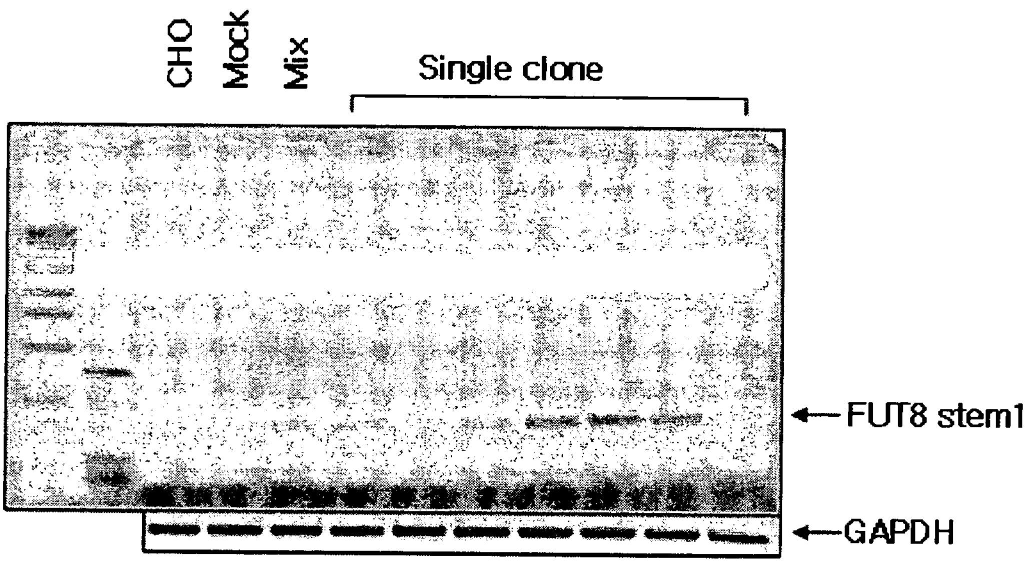
CHO
Mock
Mix
Single clone
FUT8 stem1
GAPDH

FIG. 3a

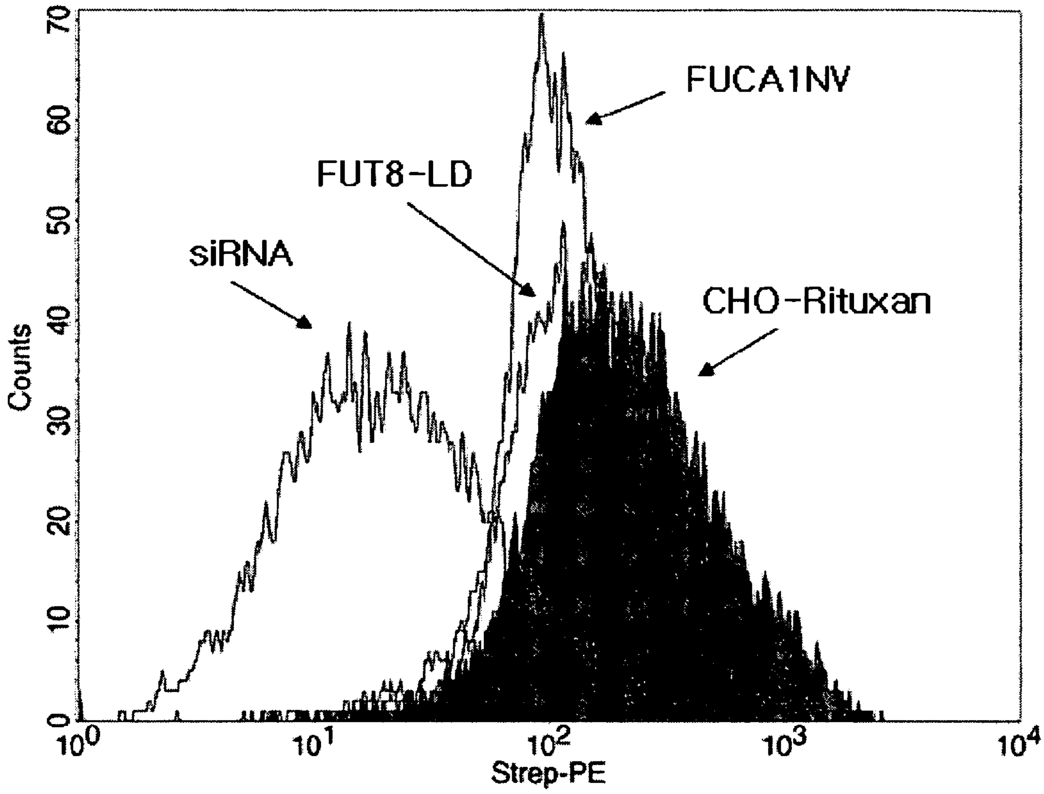
FUCA1NV
FUT8-LD
siRNA
CHO-Rituxan
Counts
0
10
20
30
40
50
60
70
$10^0$
$10^1$
$10^2$
$10^3$
$10^4$
Strep-PE

FIG. 3b

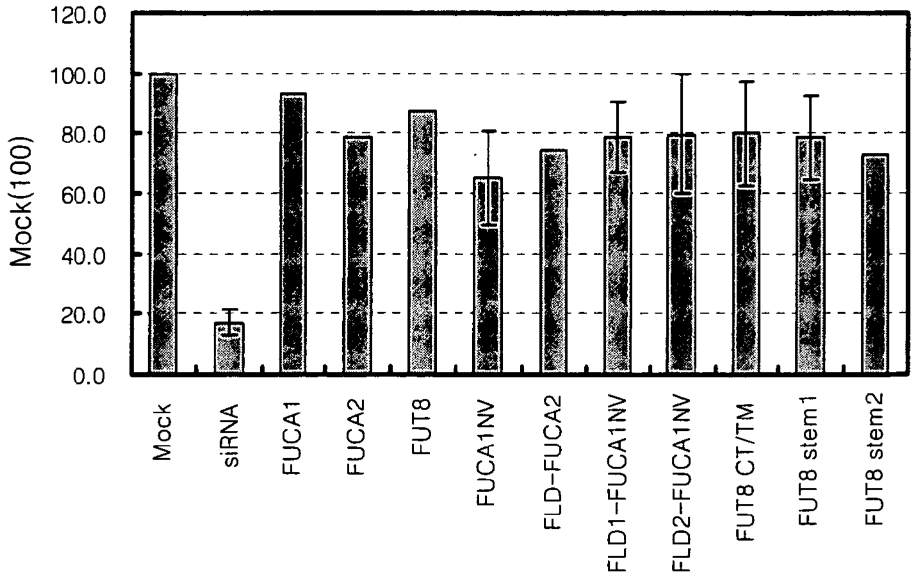
Mock(100)
120.0
100.0
80.0
60.0
40.0
20.0
0.0
Mock
siRNA
FUCA1
FUCA2
FUT8
FUCA1NV
FLD-FUCA2
FLD1-FUCA1NV
FLD2-FUCA1NV
FUT8 CT/TM
FUT8 stem1
FUT8 stem2

FIG. 4

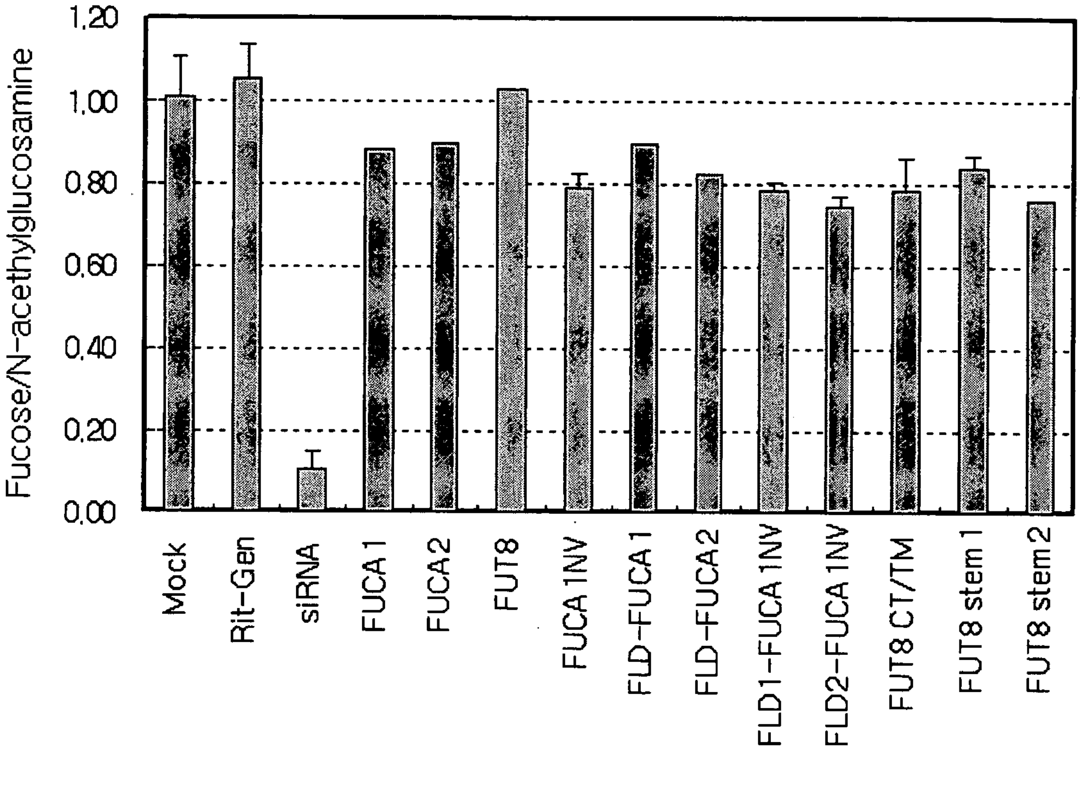
Fucose/N-acethylglucosamine
1.20
1.00
0.80
0.60
0.40
0.20
0.00
Mock
Rit-Gen
siRNA
FUCA1
FUCA2
FUT8
FUCA1NV
FLD-FUCA1
FLD-FUCA2
FLD1-FUCA1NV
FLD2-FUCA1NV
FUT8 CT/TM
FUT8 stem1
FUT8 stem2

FIG. 5a

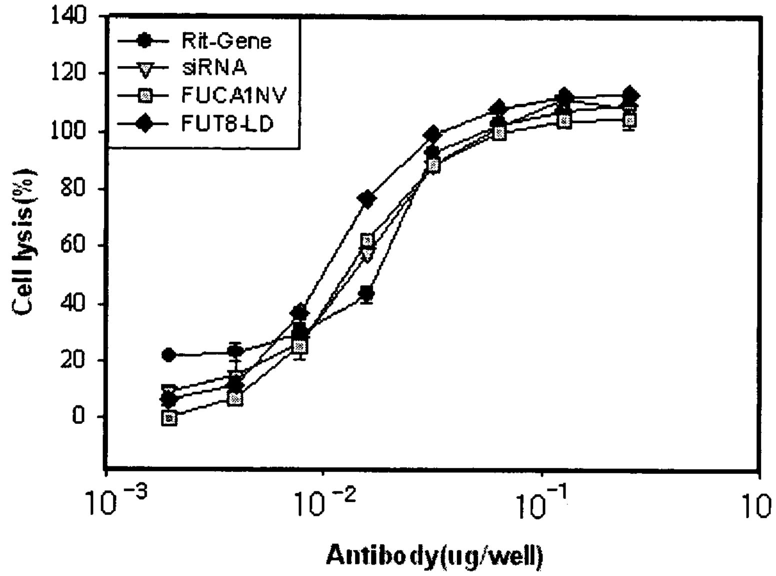
140
120
100
80
60
40
20
0
Cell lysis(%)
Rit-Gene
siRNA
FUCA1NV
FUT8-LD
$10^{-3}$
$10^{-2}$
$10^{-1}$
10
Antibody(ug/well)

FIG. 5b

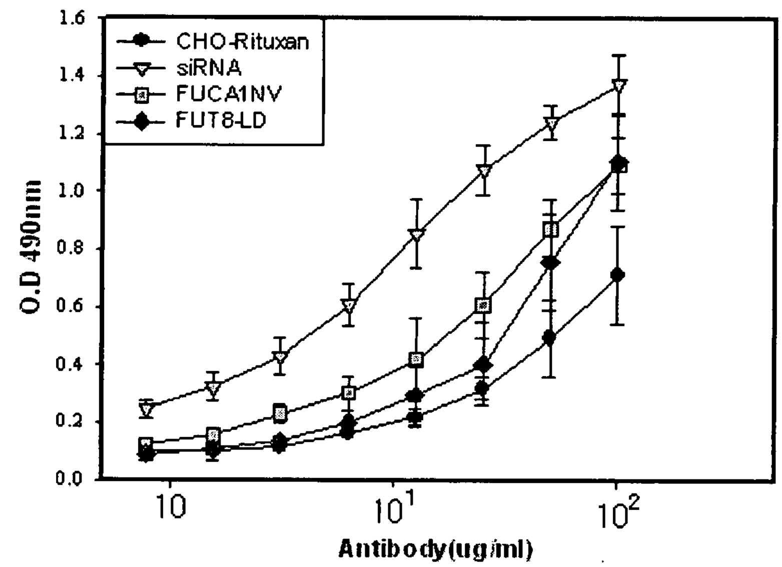
1.6
1.4
1.2
1.0
0.8
0.6
0.4
0.2
0.0
O.D 490nm
CHO-Rituxan
siRNA
FUCA1NV
FUT8-LD
10
$10^1$
$10^2$
Antibody(ug/ml)

FIG. 5c

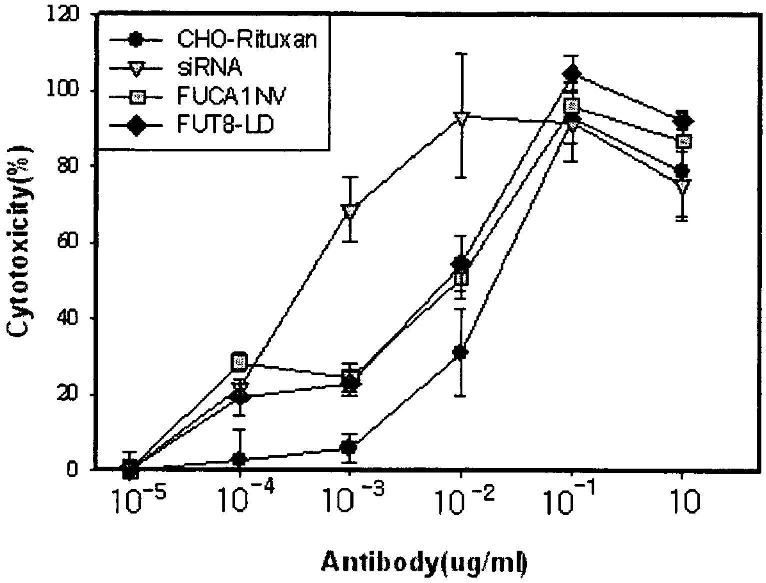
120
100
80
60
40
20
0
Cytotoxicity(%)
CHO-Rituxan
siRNA
FUCA1NV
FUT8-LD
$10^{-5}$
$10^{-4}$
$10^{-3}$
$10^{-2}$
$10^{-1}$
10
Antibody(ug/ml)

Europäisches Patentamt
European Patent Office
Office européen des brevets

# EUROPEAN SEARCH REPORT

Application Number
EP 09 00 0153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | EP 1 176 195 A (KYOWA HAKKO KOGYO KK [JP]) 30 January 2002 (2002-01-30)<br>* claims 1-62; examples 5,8 *<br>----- | 1-7,13, 14 | INV.<br>C12N15/62<br>C12N9/24<br>C12N9/10<br>C07K16/18 |
| Y | WO 94/14472 A (UNIV CALIFORNIA [US]; CARSON DENNIS A [US]; WICKS IAN [AU]) 7 July 1994 (1994-07-07)<br>* abstract; sequences 1,2 *<br>----- | 1-7,13, 14 | |
| Y | DATABASE UniProt [Online] 1 June 2001 (2001-06-01), "RecName: Full=Plasma alpha-L-fucosidase; EC=<A HREF="http://srs.ebi.ac.uk/srsbin/cgi-bin/wgetz?[enzyme-ECNumber:3.2.1.51]+-e">3.2.1.51</A>; AltName: Full=Alpha-L-fucoside fucohydrolase 2; Short=Alpha-L-fucosidase 2; Flags: Precursor;"<br>XP002533035<br>retrieved from EBI accession no. UNIPROT:Q9BTY2<br>Database accession no. Q9BTY2<br>* the whole document *<br>----- | 1-7,13, 14 | **TECHNICAL FIELDS SEARCHED (IPC)**<br>C12N<br>C07K |
| A | EP 1 500 698 A (KYOWA HAKKO KOGYO KK [JP]) 26 January 2005 (2005-01-26)<br>* page 402; claims 1-11; sequences 3,4 *<br>----- | 1,15 | |
| D,A | US 2004/110704 A1 (YAMANE NAOKO [JP] ET AL) 10 June 2004 (2004-06-10)<br>* claims 1-43; examples 1-7 *<br>-----<br>-/-- | 1,15 | |

The present search report has been drawn up for all claims

1

| Place of search | Date of completion of the search | Examiner |
|---|---|---|
| The Hague | 19 June 2009 | Gurdjian, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 00 0153

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | AVILÉS M ET AL: "Immunocytochemical localization and biochemical characterization of a novel plasma membrane-associated, neutral pH optimum alpha-L-fucosidase from rat testis and epididymal spermatozoa." THE BIOCHEMICAL JOURNAL 15 SEP 1996, vol. 318 ( Pt 3), 15 September 1996 (1996-09-15), pages 821-831, XP002533034 ISSN: 0264-6021 ----- | 1-7,13, 14 | |

TECHNICAL FIELDS SEARCHED (IPC)

The present search report has been drawn up for all claims

| Place of search | Date of completion of the search | Examiner |
|---|---|---|
| The Hague | 19 June 2009 | Gurdjian, Didier |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 00 0153

This annex lists the patent family members relating to the patent documents cited in the above-mentioned European search report.
The members are as contained in the European Patent Office EDP file on
The European Patent Office is in no way liable for these particulars which are merely given for the purpose of information.

19-06-2009

| Patent document cited in search report | Publication date | Patent family member(s) | Publication date |
|---|---|---|---|
| EP 1176195 A | 30-01-2002 | AU 3672800 A | 14-11-2000 |
| | | CA 2369292 A1 | 19-10-2000 |
| | | WO 0061739 A1 | 19-10-2000 |
| | | US 2005272916 A1 | 08-12-2005 |
| WO 9414472 A | 07-07-1994 | NONE | |
| EP 1500698 A | 26-01-2005 | AU 2003236020 A1 | 20-10-2003 |
| | | CA 2481656 A1 | 16-10-2003 |
| | | WO 03085102 A1 | 16-10-2003 |
| | | US 2004110282 A1 | 10-06-2004 |
| US 2004110704 A1 | 10-06-2004 | AU 2003236022 A1 | 20-10-2003 |
| | | BR 0309145 A | 01-02-2005 |
| | | CA 2481657 A1 | 16-10-2003 |
| | | CN 1930288 A | 14-03-2007 |
| | | EP 1498485 A1 | 19-01-2005 |
| | | WO 03085107 A1 | 16-10-2003 |
| | | MX PA04009924 A | 01-07-2005 |
| | | US 2005216958 A1 | 29-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040110704 A
- US 20040093621 A
- US 6602684 B
- US 5736137 A
- KR 408844


### Non-patent literature cited in the description


- **Jain, M. et al.** *Trend in Biotechnology,* 2007, vol. 25 (7), 307-316
- **Lazar, G. A. et al.** *PNAS,* 2006, vol. 103 (11), 4005-4010
- **Shuster, M. et al.** *Cancer Research,* 2005, vol. 65 (17), 7934-7941
- **Yamane-Ohnuki, N. et al.** *Biotechnology and Bioengineering,* 2004, vol. 87 (5), 614-622
- **Roque, A. C. et al.** *, Biotechnology Progress,* 2004, vol. 20 (3), 639-654
- **Shields, R. L. et al.** *The Journal of Biological Chemistry,* 2002, vol. 277 (30), 26733-26740
- **Mori, K. et al.** *Biotechnology and Bioengineering,* 2004, vol. 88 (7), 901-908
- **Aviles, M. et al.** *Biochemical journal,* 1996, vol. 318, 821-831
- **Milland, J. et al.** *The Journal of Biological Chemistry,* 2002, vol. 277 (12), 10374-10378
- **Nilsson, T. et al.** *The EMBO Journal,* 1994, vol. 13 (3), 562-574
- **Hodoniczky J. et al.** *Biotechnology Progress,* 2005, vol. 21 (6), 1644-1652
- **Clémenceau, B.** *Blood,* 2006, vol. 107 (12), 4669-4677
- **Shinkawa, T. et al.** *Journal of Biological Chemistry,* 2003, vol. 278 (5), 3466-3473